# EUROPEAN PATENT SPECIFICATION

(11) **EP 4 041 063 B1**
(45) Date of publication and mention of the grant of the patent: **24.07.2024**
(21) Application number: 20800716.1
(22) Date of filing: 07.10.2020
(51) Int. Cl.: A61B 5/00, G06T 7/00, A61B 5/11

(54) **DISPLAY DEVICE FOR DISPLAYING SUB-SURFACE STRUCTURES AND METHOD FOR DISPLAYING SAID SUB-SURFACE STRUCTURES**
ANZEIGEVORRICHTUNG ZUR DARSTELLUNG VON SUBOBERFLÄCHENSTRUKTUREN UND VERFAHREN ZUR DARSTELLUNG DIESER SUBOBERFLÄCHENSTRUKTUREN
DISPOSITIF D'AFFICHAGE POUR L'AFFICHAGE DE STRUCTURES DE SOUS-SURFACE ET PROCÉDÉ D'AFFICHAGE DESDITES STRUCTURES DE SOUS-SURFACE

(30) Priority: 08.10.2019 IT 201900018230
(43) Date of publication of application: 17.08.2022
(73) Proprietor: Zoeen S.r.l., 27100 Pavia (IT)
(72) Inventor: TORRICELLI, Gionatan, 50144 Firenze (IT); ZERBINATI, Nicola, 27100 Pavia (IT); FACCHINI, Rodolfo, 73039 Tricase (Lecce) (IT); ZERBINATI, Umberto, 27100 Pavia (IT)
(74) Representative: Serravalle, Marco
(86) International application number: PCT/IB2020/059397
(87) International publication number: WO 2021/070067

(56) References cited:
- EP-A2- 0 867 830
- WO-A1-2008/130903
- WO-A1-2019/178287
- US-A1- 2003 108 223

## Description

This invention relates to a device for displaying sub-surface structures and a method for displaying said sub-surface structures of the type specified in the preamble of the first claim.

In particular, this invention relates to an assistive device, for example for doctors and cosmetic surgeons, designed to display internal, or subcutaneous, portions of the human body so as to facilitate the performance of at least minimally invasive operations.

Similar devices are described within patent documents US-A-2003/108223, EP-A-0867830 and WO-A-2008/130903. WO2019178287 A1 discloses wearable glasses which can display a virtual patient's anatomy.

As is well known, one of the main applications in the field of medical assistive devices for minimally invasive procedures using needles and catheters is related to the detection and display of surface venous vessels through the use of NIR illumination in the 740-850 nm band. In fact, in the wavelength window mentioned above, there is a peak absorption of deoxygenated blood that enables the detection of surface venous vessels positioned on average within the first 5 mm of depth. There are multiple display systems to assist in inserting needles and peripheral venous catheters in patients with complex clinical conditions or with features that make these surface accesses complicated, for example in children under 3 years of age or obese patients.

In addition, various display solutions can be found among the devices on the market. For example, there are well known projection display systems that project directly onto the patient's skin, such as the *Vein Viewing System AV400* devices marketed by *Accuvein* and *VeinViewei*^{®} *Flex* devices marketed by *Christiemed*, and display systems on accessory screens, whether worn or not, such as *Veinsite*^{®} devices marketed by *Vuetek Scientific* and *Eyes-On Glasses 3.0* devices marketed by *Sahaj Interactive Solutions.*

The operation principle of said systems is linked, in particular, to lighting with NIR light and the subsequent capture of images that are processed to highlight the venous structures and reproduce what is processed at the output, i.e. directly on the skin in projection systems or on a screen in the rest of the systems.

The prior art described here comprises some significant drawbacks.

In particular, this solution has clear limits in terms of detection depth and cannot be used for PICC (Peripherally Inserted Central Catheter) and Midline type devices where the use of the acoustic guide due to the depth of the required access points is mandatory. It can only be applied mainly where very superficial accesses are concerned and on large vessels, since the differential absorption, between the vessel and the regions outside the vessel, must be significant.

Applying these techniques on the face, where the depths are in any case compatible with these values, there are also significant problems associated with: the presence of reflective bones right near the vessels in many places; the small size of some vessels, however significant; and the non-planarity of the surfaces that make optimising the lighting problematic.

Due to these, and other problems, the direct vessel detection approach is particularly problematic.

In addition to this, even solely in terms of administering fillers for cosmetic medicine and, in particular, hyaluronic acid or botulinum toxin, we know that the structures of greatest concern are the arterial ones and not the venous ones.

Finally, for applications in which the target tissues are deeper than 1 cm, it is not possible to use the prior art mentioned above because the indicated threshold determines the theoretical maximum limit for light penetrating tissues due to the phenomenon of reflection on the horny layer and, above all, the phenomenon of dermal diffusion.

In this context, the technical task underlying this invention is to devise a device for displaying sub-surface structures and a method for displaying said sub-surface structures that is capable of basically overcoming at least some of the above-mentioned drawbacks.

In the context of said technical task, it is an important purpose of the invention to obtain a display device for sub-surface structures and its method for displaying that enables the subcutaneous structure, even at depths greater than 1 cm, to be displayed.

Another important purpose of the invention is to create a device and corresponding display method that enables the subcutaneous structure, including arterial vessels and not only large veins, to be displayed.

In conclusion, another purpose of the invention is to obtain a display device and corresponding method that enables the reproduction of a subcutaneous structure even when it is in hidden, for example under bone or muscle structures.

The technical task and specified purposes are achieved with a device for displaying subcutaneous structures and a method for displaying said subcutaneous structures as claimed in the appended claims **1** and **8**, respectively.

Preferred technical solutions are set forth in the dependent claims.

The features and advantages of the invention will be apparent from the detailed description of preferred embodiments of the invention, with reference to the accompanying drawings, in which:

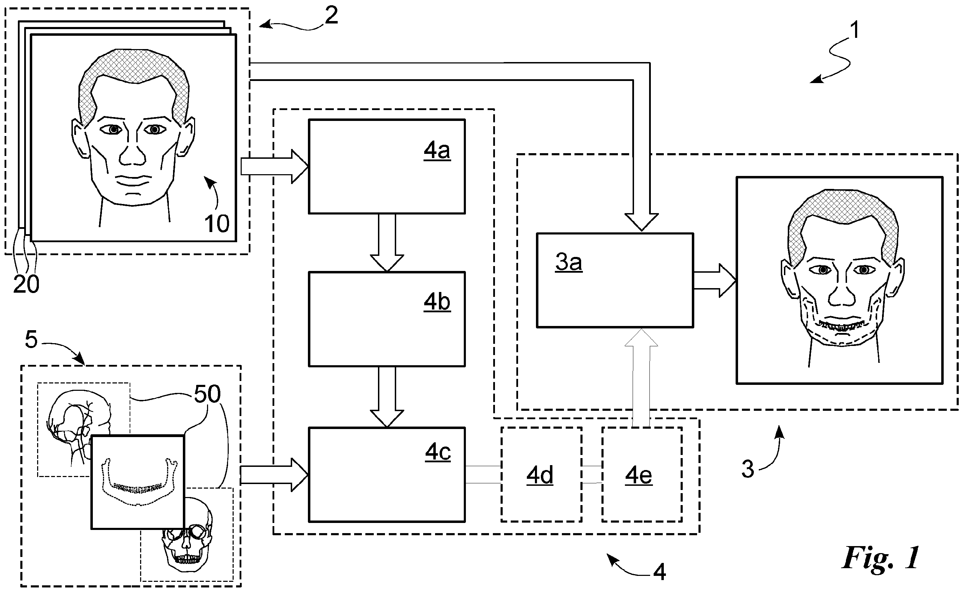

**Fig. 1** shows a functional diagram of a device for displaying sub-surface structures and a method for displaying said sub-surface structures according to the invention;

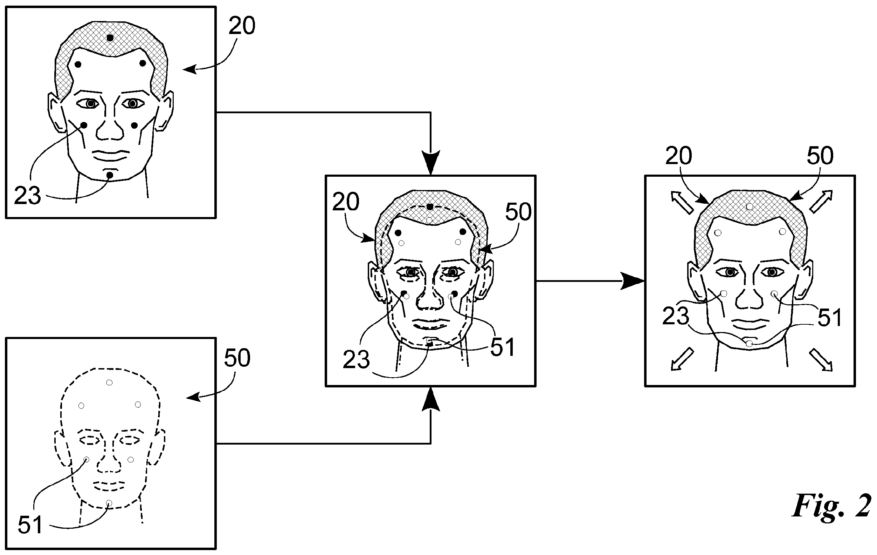

**Fig. 2** illustrates an example of adjusting a model to an image inside a device for displaying sub-surface structures and a method for displaying said sub-surface structures according to the invention wherein the overlap between the first and second localization points is shown;

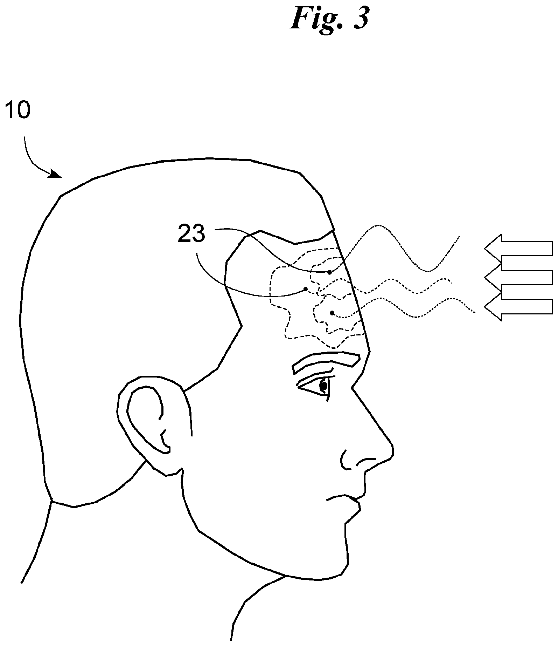

**Fig. 3** shows a diagram of the acquisition of the first localization points from different wavelength acquisition signals of a device for displaying sub-surface structures and a method for displaying said sub-surface structures according to the invention; and

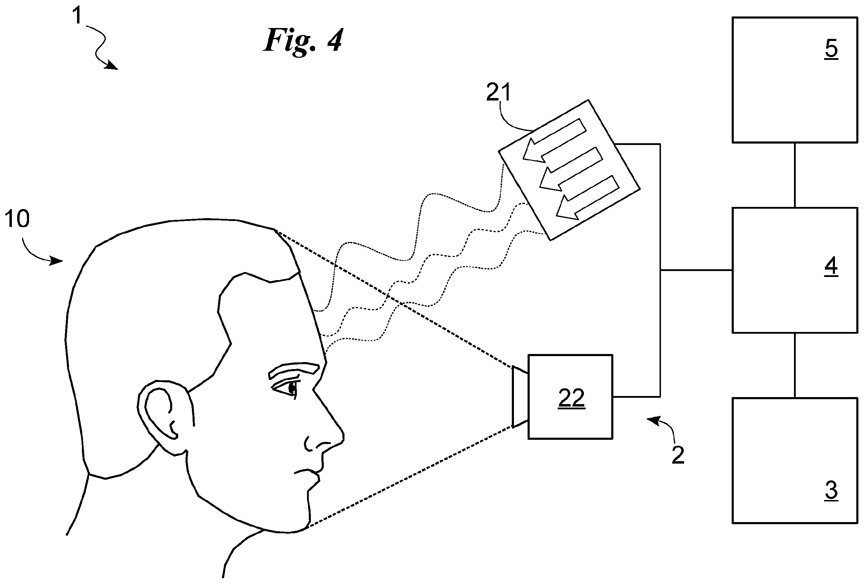

**Fig. 4** shows a functional diagram of a device for displaying sub-surface structures according to the invention in which the lighting and vision devices are shown.

In this document, the measures, values, shapes and geometric references (such as perpendicularity and parallelism), when used with words like "about" or other similar terms such as "approximately" or "substantially", are to be understood as except for measurement errors or inaccuracies due to production and/or manufacturing errors and, above all, except for a slight divergence from the value, measure, shape or geometric reference with which it is associated. For example, if associated with a value, such terms preferably indicate a divergence of no more than 10% from the value itself.

Furthermore, when terms such as "first", "second", "upper", "lower", "main" and "secondary" are used, they do not necessarily identify an order, relationship priority or relative position, but they can simply be used to distinguish different components more clearly from one another.

Unless otherwise specified, as is apparent from the following discussion, terms such as "processing", "computer science", "determination", "calculation" or similar are considered to refer to the computer action and/or processes or similar electronic computing devices that manipulate and/or transform data represented as physical, such as electronic quantities of registers of an information system and/or memory, other data similarly represented as physical quantities within computer systems, registers or other devices for storing, transmitting or displaying information.

Unless otherwise stated, the measurements and data reported in this text shall be considered as performed in International Standard Atmosphere ICAO (ISO 2533:1975).

With reference to the figures, reference number **1** globally denotes the device for displaying sub-surface structures according to the invention.

The device 1 is preferably used to assist doctors and cosmetic surgeons during invasive or minimally invasive operations, such as filler infusion steps for fillers such as botulinum toxin and/or hyaluronic acid, for example on a user's face **10.**

The device 1, of course, can equally be used in other fields, including non-human applications. The user 10 could also be a non-human living being or it could even be an inanimate object.

In any case, the invention is described in the preferred embodiment so as to highlight its relative advantages in the technical field.

In particular, the device 1 enables indications to be acquired regarding the sub-surface or subcutaneous structure of at least one part of the body.

In this case, the body part is, preferably, the face, but could also be another area, for example, the abdomen, neck, limbs, or pelvis.

Even more specifically, the term "subcutaneous structures" refers to the fact that, when used on the face of a user or even on another anatomical region, the device 1 enables the display of, for example, veins, arteries, nerves, and even muscles.

The presence and conformation of these subcutaneous structures, in fact, provides important indications for inserting needles and peripheral venous catheters, enabling them to be directed appropriately.

As already mentioned, the same technique could also assist the operators in other procedures (not falling within the scope of the claimed method) for administering drugs and forms of energy so as to optimise the procedures and the effects thereof. In fact, the device 1 could be used for inserting Midline peripheral venous catheters, PICCs, and Trocars, for laparoscopy operations, as well as needles connected to electromedical tools that perform local, minimally invasive operations such as, merely by way of example, radiofrequency or optical sources.

The device 1, for these purposes, comprises acquisition means **2** and display means **3.**

The acquisition means 2 are configured to acquire images **20** of at least part of the body of the user 10 or of an object. The acquisition means 2 are preferably designed to acquire two-dimensional images 20.

In order to acquire these images 20, the acquisition means preferably include a lighting device **21** and a vision device **22.**

The lighting device 21 is preferably configured to light at least part of the body of the user 10 or of the object. The illumination of this body part preferably occurs via acquisition signals defining a multispectral radiation band that can be predetermined.

The phrase "that can be predetermined" refers to the fact that the radiation band can be either predetermined, for example when the lighting device 21 emits a fixed radiation spectrum, or if the lighting device 21 can coincide with the environmental lighting, for example, solar or controlled light.

The lighting device 21 basically illuminates the user 10 with different types of optical radiation, within the selected band, to detect the images 20 across the spectral field of the examination.

In fact, the lighting device 21 enables the acquisition of a plurality of images 20 of the body part inside the band concerned.

In particular, the term "multispectral radiation band" refers to the fact that the acquisition signals are optical signals emitted at different wavelengths within a pre-set band. In fact, the operator can control the band and predetermine the emission wavelengths, including based on the body part concerned.

Different wavelength signals entail different tissue responses with consequent different characteristics detected and image depths equally diversified.

Even more specifically, the acquisition signals are, preferably, of the NIR type, or Near InfraRed. With reference to the above, it is possible to arrange it so that, while from any ultraviolet and visible images 20 you can obtain useful information on the purely surface structures, from the visible red bands, as well as from the NIR bands, you can obtain information on layers slightly deeper and difficult to see with the naked eye, like, for example, the main veins or other information associated with the fat layers and deeper tissues.

The vision device 22 is preferably optical and designed to record the images 20 of the body of the user 10 and of the object highlighted by the lighting device 21.

The vision device 22 thus enables the acquisition means 2 to obtain images 20 of at least part of the body of the user 10 or of the object, by cooperating with the lighting device 21. In particular, the devices 21, 22 can cooperate according to different modes - modes that are known in the current state of the art.

For example, the lighting device 21 can strike the part of the body of the user 10 or of the object with a continuous multispectral light signal and vision means 22 can acquire selected images at different wavelengths of the light spectrum. Alternatively, for example, the lighting device 21 and the vision device 22 may be synchronised and the lighting device 21 may emit discontinuous light signals, or flashes, at different wavelengths acquired by the vision device 22 each time.

In any case, the acquisition means 2 enable the acquisition of each of the images 20 at a corresponding wavelength, in the same moment in time or in successive moments in time, preferably close together. Alternatively, they could be arranged so that they acquire a plurality of images 20 in the same moment and at the same wavelength with the effect described below. Or, again, an acquisition of a plurality of images 20 in the same moment at different wavelengths and different points of view.

As can be seen, more than the method of acquiring images 20, it is important to have the possibility of acquiring at least one image 20 at a predetermined wavelength and, to be able to reconstruct a genuine vector containing a plurality of images 20 and, a plurality of images 20 at different wavelengths, whether acquired in the same moment in time or in successive moments in time.

The display means 2 are, on the other hand, configured to make at least one of the images 20 accessible to an operator. This image 20 is preferably the first image 20 acquired, or the surface image of the user 10 or of the object. In addition, the image 20 is displayed in real time. However, a storage device for images could also be included so as to enable subsequent consultation.

For example, therefore, the display means 3 could also comprise a simple screen, whether worn or not, designed to enable the display of the images acquired, preferably in a digital form.

Preferably, therefore, the light means 2 and the display means 3 are operationally connected together.

In particular, the device 1 comprises a processor 4. The processor 4 is designed to connect the acquisition means 2 and the display means 3. In addition, the processor 4 is configured to coordinate the acquisition means 2 and the display means 3. If necessary, the processor 4 can also be set up so as to coordinate the devices 21, 22.

Basically, especially when the transmission of the images is simultaneous, or streamed, the processor 4 enables the correct display of the successive images 20 on the display means 3.

Advantageously, the processor 4 is also configured to extract the images 20 from the reference signals.

The reference signals preferably include first surface localization points **23** that are defined by the body part or the object. In other words, each body part defines particular localization points that enable the conformation itself of the body part being examined to be determined.

The first localization points 23 preferably match, in fact, the landmarks.

The landmarks are points used to localise a region of the body in a unique manner, without any confusion. These points are, therefore, anatomical and basically identify, in a unique manner, the different body parts so as to enable the positions of these points, and the connected areas of concern, to be returned to, as needed, always making reference to these fixed, general, and shared points.

Since the images 20 derive from acquisition signals at different wavelengths, the first localization points 23 basically include both the identified points on the surface of the body part or object, and the sub-surface projections of these points inside the same body part or object. Basically, therefore, the reference signals, more precisely, include a matrix of first localization points 23 in which the localization points are detected at different wavelengths. Basically, this matrix defines a two-dimensional mapping at different wavelengths, or different depths, of the body part or object. The first localization points 23 are, therefore, fixed points that can be identified on the tissue of the user 10 capable of enabling a basic surface mapping and, if required, also a sub-surface mapping of the tissue itself.

The device 1, according to the invention, comprises a database 5 as well.

The database 5 can be arranged inside the physical memory supports, inside or outside the processor 4, or it can be included inside the remote platforms that are accessible via cloud systems.

In this respect, the processor 4 may include the database 5 or include physical or wireless connection means, for communicating with the database 5.

The connection systems of this type, such as USB, Ethernet, Bluetooth, Wi-Fi, or the like are well known in the current state of the art are not the special subject of this invention.

The database 5 is operationally connected to the processor 4.

The database 5 includes at least one sub-surface structure model 50 of the subject subjected to the acquisition means 2. For example, if the subject is a body part of the user 10, the database includes at least one subcutaneous structure model 50 of the body part concerned.

The database 5, in addition, comprises a plurality of sub-surface structure models 50, for example subcutaneous ones, of the body part or object. Of course, the database 5 could comprise, and preferably does comprise, a plurality of models 50 referring to all the body parts.

The database 5 preferably comprises a plurality of sub-surface structure models 50 referring to different depths of the body parts of the user 10 or the object. For example, the models 50 may include facial muscles, or face veins, or nerves and, therefore, define different configurations at different depths potentially of the same body part.

The models 50 are, of course, anatomic models that are predetermined and, for the most part, known in the current state of the art.

Each of the models 50, defines a predetermined configuration of second localization points **51.**

The second localization points 51 are, like the first localization points 22, points that identify predetermined body parts. The second localization points 51 may also consist in landmarks that, in any case, refer to the models 50 and not to the images 20 acquired relating to the user 10.

The plurality of models present in the database 5 makes it possible to determine in a rather precise manner the sub-surface conformation of the user 10 or object. In fact, it is possible to compare the conformation of the body part of the user 10, determined with the first localization points 23, with the conformation of each model 50, determined with the second localization points 51.

The processor 4 is configured to compare the model 50, or each of the models 50, with the reference signals of at least one image 20 and to select the model 50 the second localization points 51 of which most closely match the first localization points 23.

Since the reference signals are preferably defined by a matrix of first localization points 23 at different wavelengths, deriving from images 20 at different wavelengths corresponding to different depths of the body or object, the processor 4 selects, for example, the first localization points 23 at the same wavelength and compares them with the second localization points 51 of the models 50.

Of course, the first localization points 23 and the second localization points 51 may not coincide perfectly. For this reason, the processor 4 cross-references the data of the images 20 and the models 50 and performs a "matching" of this information to identify the most similar model 50 and, following this, implements an adjustment, or stretching, of the same model 50 so as to make the first localization points 23 and the second localization points 51 coincide.

The processor preferably adjusts a selected model 50 to a selected image 20. "Adjustment" refers to the fact that, based on known algorithms in the state of the art, the processor 4 deforms the model 50 so as to perfectly overlap the localization points 23, 51 of the image 20 and of the model 50 respectively.

Basically, therefore, the determination of the model 50 has a basis that is mainly based on probability or statistics. However, it has been observed that this basis is enough to determine, in a current and functional manner, the subcutaneous structures of the body part concerned.

In addition to the above, the display means 3 are configured to make the selected model 50 also accessible to the operator in real time so that the operator can see the subcutaneous structures of the body part of the user 10.

The models 50 stored in the database 5 can be models with various different characteristics and of various tissues of different body parts of the user 10. For example, the models 50 may have the configurations of veins, or of arteries, or of nerves present on the face of a user 10, or the configurations at different depths or according to different second localization points 51.

These models 50 can, in examples not covered by the claims, be two-dimensional. The models 50 according to the invention are three-dimensional. In the latter case, each of the images 20, which is preferably two-dimensional, is acquired at a respective wavelength, which is different from the others in the same moment or in successive moments, by acquisition signals so that, by reconstructing a vector determined by set of images, three-dimensional reference signals are created, including a matrix of first localization points, defining a three-dimensional mapping of the body part concerned.

Therefore, the reconstruction of the vector of images 20 can enable a comparison of the two-dimensionally derived signals, with second localization points 51 defined by the three-dimensional models.

Alternatively to the above, as already mentioned, the acquisition means 2 could in embodiments not covered by the claims acquire a plurality of images 20 in the same moment of time and at the same wavelength, possibly from different positions, of the acquisition signals, so as to reconstruct images 20 of part of the body in three dimensions. In this latter case, the first localization points 23 are already determined in a three-dimensional space and the processor 4 performs a comparison, or matching, of the images 20 and of the second localization points 51 in a three-dimensional environment.

The display means 3 reproduce both the images 20 and the selected model 50.

This display can be separated. The display means 3 preferably reproduce, simultaneously, the images 20 and the model 50 selected overlapping the model 50 with the images 20 in all the moments in time. More specifically, the display means 3 simultaneously reproduce the images 20 in real time, preferably each surface image 20 in the successive moments in time, and a model 50 adjusted as needed, in real time, to the images 20 displayed.

The images 20 and the model 50 selected can, therefore, be reproduced in two dimensions by the display means 3, or they can also be reproduced in three dimensions. The latter configuration can also be adopted, above all when the display means 3 are, for example, augmented reality glasses, such as Oculus or the like. Preferably, as mentioned, the processor 4 extrapolates the first image 20 acquired by the acquisition means 2 in each moment in time, or the surface image of the body part of the user 10 or the object, and overlaps the model 50 selected and adjusts this image 20 on the display means 3.

The operation of the display device 1 described in structural terms above is the following.

The lighting device 21, if present, is oriented towards the area or body part of the user 10 or the object concerned so as to frame it and enable radiation to strike this area. At the same time, the vision device 22 records the images 20 determined by the lighting device 21 or by the environmental lighting.

The operator is, therefore, able to display the images 20, preferably, but not exclusively, each first image 20 of each moment in time, acquired via the acquisition means 2 thanks to the display means 3. The display means 3 show the operator at least one image 20 of the body part of the user 10, preferably the surface image 20, on which the selected model 50 is superimposed based on the correspondence between the localization points 23, 51. The operator can preferably consult the images 20 in real time, or streaming, while he or she moves the acquisition means 2, obtaining the image 20 of the patient on the display means 2 on which the selected model 50 is also transparently projected.

The invention comprises a new method for displaying sub-surface structures.

The method is preferably implemented with the device 1.

In any case, the method, in essence, preferably comprises an acquisition step 4a, a processing step 4b, a comparison and selection step 4c, and a display step 3a. In the acquisition step 4a, preferably, a plurality of images 20 is acquired of a body part of the user 10 via the acquisition signals defining a multispectral frequency band.

The acquisition step 4a, in particular, occurs illuminating the body part of the user 10 of the object with the lighting device 21, recording the images 20 deriving from the lighting via the vision device 22, and forwarding these images 20 to the processor 4.

In the processing step 2b, the reference signals, which include the first localization points 23, are extracted from the images.

In the comparison and selection step 4c, at least one image 20 is compared with a plurality of subcutaneous structure models 50 of the body part. As already described, each of the models 50 defines predetermined configurations of the second localization points 51.

In addition, from the comparison, the model 50 the second localization points 51 of which best correspond to the first localization points 23 is selected.

As already mentioned, the first localization points 23 basically correspond to the landmarks relating to the user 10, or to the images 20, and, in the same way, the second localization points 51 are landmarks arranged on the models 50.

In conclusion, in the display step 3a, preferably, the images 20 and the selected model 50 are reproduced or displayed in such a way that the operator can see the sub-surface structure of the body part of the user 10.

As already mentioned, to functionally display the sub-surface structures, the processor 4 preferably performs an adjustment step **4d,** moment by moment, adjusting the model 50 to the images 20 that follow in each moment.

In fact, in particular, the images 20 and the selected model 50 are preferably, but not necessarily, displayed simultaneously, superimposing the model 50 over the images 20 in each moment in time.

In particular, the processor 4 adjusts, in the adjustment step 4d that occurs during or prior to the display step 3a, the model 50, deforming it in such a way that the second localization points 51 coincide with the first localization points 23.

Following this adjustment, the processor 4 preferably implements a superimposing or combination step **4e.**

In the superimposing step 4e, the processor 4 preferably combines the selected image 20, preferably the first surface image 20 transposed, with the selected and adjusted model 50. The processor 4, in addition, if required, could also process transparency effects or other effects or deformations, including based on the operator's commands.

As a consequence, the display means 3 display, in the display step 3a, the first image 20 and, preferably, the selected model 50, adjusted and superimposed on the image 20 transposed by acquisition signals to the wavelength concerned; as already fully explained, the image 20 corresponds to the surface representation, in the same moment in time, of the same body part represented by the other images 20 at the different wavelengths, for example used to select the model 50.

In addition, the method could involve, in the acquisition step 4a, the acquisition, in the same moment in time and at the same wavelength, of acquisition signals so as to reconstruct the images 20 of the body part in three dimensions.

In conclusion, the selection of the acquisition signal, or of the wavelength concerned, can be pre-set, for example with reference to the type of model 50 that is to be displayed, or can also be manually set by the operator. In general, the processor 4 can include control means designed to enable the operator to select the type of model 50 concerned. For example, the operator can choose to display the nerves of the face and the processor 4 can be set to control the selection of images 20 corresponding to the wavelength to which the tissues have penetrated at the correct depth to display these nerves.

The device 1 for displaying sub-surface structures according to the invention entails important advantages.

In fact, the device makes it possible to display subcutaneous structures at depths greater than 1 cm and even when the structures are arranged, for example, below the interference elements such as bone or muscle structures since it is based on complete, predetermined models and on real observations only in certain points.

In this sense, one big advantage of the device 1 is that it can also display internal bodies, such as the vein branches on the face of a user 10, which are not immediately visible at the surface, as is the case, in contrast, for the veins of the superior arteries.

These features entail the significant advantage of helping the operator during invasive or minimally invasive operations, enabling them to localise the portions of the body concerned and correctly direct any intracutaneous tools.

In fact, the device 1 makes it possible to avoid damage owing to injections of botulinum toxin and/or hyaluronic acid inside particular arteries that must not be touched, such as, for example the angular arteries.

In addition, the chance of puncturing vessels, both arteries and veins, is significantly reduced, if not eliminated. Such punctures, when large, may generate hematomas that can remain visible for days and also cause pain and irritation.

In addition, it is possible to reduce damage to nerve tissue that can be the cause of particular pain.

In conclusion, the display of the mutual position of muscles, bones, vessels, and nerves, can give an indication of the optimal points and directions for insertion in order to reach the areas specifically useful for treatment.

The invention is susceptible to variations falling within the scope of the inventive concept defined by the claims.

## Claims

1. A display device (1) for displaying sub-surface structures comprising:
- acquisition means (2) configured to acquire images (20) of at least part of the body of a user (10) or of an object from acquisition signals defining a pre-determinable multispectral radiation band,
- a processor (4) configured and to extract, from such images (20), reference signals including first surface and/or sub-surface localization points (23) defined by said part of said body or said object,
- a database (5) operationally connected to said processor (4) and including a plurality of models (50) of sub-surface structures of said part of said body or said object, each defining predetermined configurations of second localization points (51),
- said processor (4) being configured to compare each of said models (50) with said reference signals and select one of said models (50) in which said second localization points (51) match more with said first localization points (23),
and said device (1) further comprising:
- display means (3) configured to make at least one of said images (20) accessible to an operator in real time and to make accessible to said operator also said model (50) selected in such a way that said operator can see, in real time, the sub-surface structure of said part of said body or said object,
and wherein:
- said processor (4) is configured to coordinate said acquisition means (2) and said display means (3), and
- said models (50) are predetermined anatomic models including, for instance, facial muscles, or face veins, or nerves so that to define different configurations at different depths potentially of the same body part,
- said models (50) are three-dimensional and each of said images (20) is acquired at a respective wavelength of said acquisition signals, the respective wavelengths of each of each of said images being different from each other, in such a way as to produce, by reconstructing a vector determined by all said images, three-dimensional reference signals defining a mapping of said part of said body.

2. The device (1) according to claim 1, in which said acquisition means (2) comprise a lighting device (21) configured to illuminate at least part of said body or said object by means of said acquisition signals in such a way as to acquire said images (20) within said band, and a vision device (22) configured to record said images (20).

3. The device (1) according to at least one preceding claim, in which said reference signals include a matrix of said first localization points (23) in which said first localization points (23) are detected at different wavelengths in such a way as to define a two-dimensional mapping at different wavelengths, or different depths, of said part of said body or said object.

4. The device (1) according to at least one preceding claim, in which said display means (3) reproduce simultaneously said images (20) and said model (50) selected by superimposing said model (50) to said images (20) during each moment of time.

5. The device (1) according to at least one preceding claim, in which said processor (4) adapts, at any moment of time, said model (50) to said images (20) by deforming said model (50) in such a way that said localization points (23, 51) coincide and said display means (3) reproduce said model (50) adapted, at any moment, to said images (20).

6. The device (1) according to at least one preceding claim, in which said localization points match with landmarks.

7. The device (1) according to at least one preceding claim, in which said acquisition means (2) acquire a plurality of images at the same moment of time and at the same wavelength of said acquisition signals in such a way as to reconstruct said part of the body in three dimensions.

8. A method for displaying sub-surface structures comprising:
- acquiring (4a) a plurality of images (20) of a part of the body of a user (10) or of an object by means of acquisition signals defining a multispectral frequency band,
- processing (4b) said images (20) to extract reference signals including first surface and/or sub-surface localization points (23) defined by said part of said body or said object,
- comparing (4c) said images (20) with a plurality of models (50) of sub-surface structures of said part of said body or said object, each defining predetermined configurations of second localization points (51),
- selecting (4c) said model (50) in which said second localization points (51) match more with said first localization points (23),
and comprising:
- displaying (3a) at least one of said images (20) and said model (50) selected in such a way that said operator can see, in real time, the sub-surface structure of said part of said body or said object,
- said models (50) being predetermined anatomic models including, for instance, facial muscles, or face veins, or nerves so that to define different configurations at different depths potentially of the same body part, and being three-dimensional and each of said images (20) is acquired at a respective wavelength of said acquisition signals, the respective wavelengths of each of each of said images being different from each other, in such a way as to produce, by reconstructing a vector determined by all said images, three-dimensional reference signals defining a mapping of said part of said body.

9. The method according to at least claim 8, in which during or before said displaying step (3a), said processor (4) adapts (4d), moment by moment, the selected model (50) to said images (20) by deforming the selected model (50) in such a way that said second localization points (51) coincide with said first localization points (23), and superimposes (4e) the selected model (50) to at least one said image (20) in such a way as to display (3a) said model (50) and said image (20) mutually combined with each other.

## Patentansprüche

1. Anzeigevorrichtung (1) zum Anzeigen von Unterflächenstrukturen, umfassend:
- Erfassungsmittel (2), die konfiguriert sind, um Bilder (20) von mindestens einem Teil des Körpers eines Benutzers (10) oder eines Objekts aus Erfassungssignalen zu erfassen, die ein vorbestimmtes multispektrales Strahlungsband definieren,
- einen Prozessor (4), der konfiguriert ist und aus solchen Bildern (20) Referenzsignale extrahiert, die erste Oberflächen- und/oder Unterflächenlokalisierungspunkte (23) enthalten, die durch den Teil des Körpers oder des Objekts definiert sind,
- eine Datenbank (5), die betriebsmäßig mit dem Prozessor (4) verbunden ist und mehrere Modelle (50) von Unterflächenstrukturen des Teils des Körpers oder des Objekts beinhaltet, die jeweils vorbestimmte Konfigurationen von zweiten Lokalisierungspunkten (51) definieren,
- der Prozessor (4) konfiguriert ist, um jedes der Modelle (50) mit den Referenzsignalen zu vergleichen und eines der Modelle (50) auszuwählen, in dem die zweiten Lokalisierungspunkte (51) mehr mit den ersten Lokalisierungspunkten (23) übereinstimmen,
und die Vorrichtung (1) ferner Folgendes umfasst:
- Anzeigemittel (3), die konfiguriert sind, um mindestens eines der Bilder (20) einem Bediener in Echtzeit zugänglich zu machen und um dem Bediener auch das Modell (50) zugänglich zu machen, das so ausgewählt ist, dass der Bediener in Echtzeit die Unterflächenstruktur des Teils des Körpers oder des Objekts sehen kann,
und wobei:
- der Prozessor (4) konfiguriert ist, um die Erfassungsmittel (2) und die Anzeigemittel (3) zu koordinieren, und
- die Modelle (50) vorbestimmte anatomische Modelle sind, einschließlich zum Beispiel Gesichtsmuskeln oder Gesichtsvenen oder Nerven enthalten, so dass unterschiedliche Konfigurationen in unterschiedlichen Tiefen möglicherweise desselben Körperteils definiert werden,,
- die Modelle (50) dreidimensional sind und jedes der Bilder (20) bei einer entsprechenden Wellenlänge der Erfassungssignale erfasst wird, wobei sich die jeweiligen Wellenlängen jedes der Bilder voneinander unterscheiden,so dass durch Rekonstruktion eines durch alle Bilder bestimmten Vektors dreidimensionale Referenzsignale erzeugt werden, die eine Abbildung des Teils des Körpers definieren.

2. Vorrichtung (1) nach Anspruch 1, bei der die Erfassungsmittel (2) eine Beleuchtungsvorrichtung (21), die so konfiguriert ist, dass sie zumindest einen Teil des Körpers oder des Objekts mit Hilfe der Erfassungssignale so beleuchtet, dass die Bilder (20) innerhalb des Bandes erfasst werden, und eine Sichtvorrichtung (22) umfassen, die so konfiguriert ist, dass sie die Bilder (20) aufzeichnet.

3. Vorrichtung (1) nach mindestens einem vorhergehenden Anspruch, bei der die Referenzsignale eine Matrix der ersten Lokalisierungspunkte (23) beinhalten, in der die ersten Lokalisierungspunkte (23) bei unterschiedlichen Wellenlängen erfasst werden, um eine zweidimensionale Abbildung bei unterschiedlichen Wellenlängen oder unterschiedlichen Tiefen des Teils des Körpers oder des Objekts zu definieren.

4. Vorrichtung (1) nach mindestens einem der vorhergehenden Ansprüche, bei der die Anzeigemittel (3) gleichzeitig die Bilder (20) und das Modell (50) reproduzieren, die durch Überlagern des Modells (50) mit den Bildern (20) während jedes Zeitpunkts ausgewählt werden.

5. Vorrichtung (1) nach mindestens einem vorhergehenden Anspruch, bei der der Prozessor (4) zu jedem Zeitpunkt das Modell (50) an die Bilder (20) anpasst, indem das Modell (50) auf eine solche Weise verformt wird, dass die Lokalisierungspunkte (23, 51) zusammenfallen und die Anzeigemittel (3) das Modell (50) reproduzieren, das zu jedem Zeitpunkt an die Bilder (20) angepasst ist.

6. Vorrichtung (1) nach mindestens einem vorhergehenden Anspruch, bei der die Lokalisierungspunkte mit Landmarken übereinstimmen.

7. Vorrichtung (1) nach mindestens einem vorhergehenden Anspruch, bei der die Erfassungsmittel (2) mehrere Bilder zum gleichen Zeitpunkt und bei der gleichen Wellenlänge der Erfassungssignale erfassen, um den Teil des Körpers in drei Dimensionen zu rekonstruieren.

8. Verfahren zum Anzeigen von Untergrundstrukturen, umfassend:
- Erfassen (4a) mehrerer Bilder (20) eines Teils des Körpers eines Benutzers (10) oder eines Objekts mittels Erfassungssignalen, die ein multispektrales Frequenzband definieren,
- Verarbeiten (4b) der Bilder (20), um Referenzsignale zu extrahieren, die erste Oberflächen- und/oder Unterflächenlokalisierungspunkte (23) enthalten, die durch den Teil des Körpers oder des Objekts definiert sind,
- Vergleichen (4c) der Bilder (20) mit mehreren Modellen (50) von Unterflächenstrukturen des Teils des Körpers oder des Objekts, die jeweils vorbestimmte Konfigurationen von zweiten Lokalisierungspunkten (51) definieren,
- Auswählen (4c) des Modells (50), in dem die zweiten Lokalisierungspunkte (51) mehr mit den ersten Lokalisierungspunkten (23) übereinstimmen,
und umfassend:
- Anzeigen (3a) mindestens eines der Bilder (20) und des Modells (50), die so ausgewählt sind, dass der Bediener in Echtzeit die Unterflächenstruktur des Teils des Körpers oder des Objekts sehen kann,
- die Modelle (50) vorbestimmte anatomische Modelle sind, die beispielsweise Gesichtsmuskeln oder Gesichtsvenen oder Nerven enthalten, um verschiedene Konfigurationen in verschiedenen Tiefen potenziell desselben Körperteils zu definieren, und dreidimensional sind und jedes der Bilder (20) bei einer jeweiligen Wellenlänge der Erfassungssignale erfasst wird, wobei die jeweiligen Wellenlängen jedes der Bilder voneinander verschieden sind, so dass durch Rekonstruktion eines durch alle Bilder bestimmten Vektors dreidimensionale Referenzsignale erzeugt werden, die eine Abbildung des Teils des Körpers definieren.

9. Verfahren nach mindestens Anspruch 8, bei dem der Prozessor (4) während oder vor dem Anzeigeschritt (3a) das ausgewählte Modell (50) Moment für Moment an die Bilder (20) anpasst (4d), indem er das ausgewählte Modell (50) so verformt, dass die zweiten Lokalisierungspunkte (51) mit den ersten Lokalisierungspunkten (23) zusammenfallen, und das ausgewählte Modell (50) mindestens einem der Bilder (20) in der Weise überlagert (4e), dass das Modell (50) und das Bild (20) miteinander kombiniert angezeigt werden (3a).

## Revendications

1. Dispositif (1) d'affichage destiné à afficher des structures de sous-surface comprenant :
- des moyens d'acquisition (2) configurés pour acquérir des images (20) d'au moins une partie du corps d'un utilisateur (10) ou d'un objet à partir de signaux d'acquisition définissant une bande de rayonnement multispectral prédéterminable,
- un processeur (4) configuré pour extraire, à partir de telles images (20), des signaux de référence comprenant de premiers points de localisation (23) de surface et/ou de sous-surface définis par ladite partie dudit corps ou dudit objet,
- une base de données (5) fonctionnellement connectée audit processeur (4) et comprenant une pluralité de modèles (50) de structures de sous-surface de ladite partie dudit corps ou dudit objet, chacun définissant des configurations prédéterminées de seconds points de localisation (51),
- ledit processeur (4) étant configuré pour comparer chacun desdits modèles (50) avec lesdits signaux de référence et sélectionner l'un desdits modèles (50) dans lequel lesdits seconds points de localisation (51) correspondent davantage auxdits premiers points de localisation (23),
et ledit dispositif (1) comprenant en outre :
- des moyens d'affichage (3) configurés pour rendre au moins l'une desdites images (20) accessible à un opérateur en temps réel et pour rendre également accessible audit opérateur ledit modèle (50) sélectionné de telle sorte que ledit opérateur puisse voir, en temps réel, la structure de sous-surface de ladite partie dudit corps ou dudit objet,
et dans lequel :
- ledit processeur (4) est configuré pour coordonner lesdits moyens d'acquisition (2) et lesdits moyens d'affichage (3), et
- lesdits modèles (50) sont des modèles anatomiques prédéterminés comprenant, par exemple, des muscles du visage, ou des veines du visage, ou des nerfs afin de définir différentes configurations à différentes profondeurs potentiellement de la même partie de corps,
- lesdits modèles (50) sont tridimensionnels et chacune desdites images (20) est acquise à une longueur d'onde respective desdits signaux d'acquisition, les longueurs d'onde respectives de chacune de chacune desdites images étant différentes les unes des autres, de manière à produire, par reconstruction d'un vecteur déterminé par toutes lesdites images, des signaux de référence tridimensionnels définissant une cartographie de ladite partie dudit corps.

2. Dispositif (1) selon la revendication 1, dans lequel lesdits moyens d'acquisition (2) comprennent un dispositif d'éclairage (21) configuré pour éclairer au moins une partie dudit corps ou dudit objet au moyen desdits signaux d'acquisition de manière à acquérir lesdites images (20) dans ladite bande, et un dispositif de vision (22) configuré pour enregistrer lesdites images (20).

3. Dispositif (1) selon au moins une revendication précédente, dans lequel lesdits signaux de référence comprennent une matrice desdits premiers points de localisation (23) dans laquelle lesdits premiers points de localisation (23) sont détectés à des longueurs d'onde différentes de manière à définir une cartographie bidimensionnelle à des longueurs d'onde différentes, ou à des profondeurs différentes, de ladite partie dudit corps ou dudit objet.

4. Dispositif (1) selon au moins une revendication précédente, dans lequel lesdits moyens d'affichage (3) reproduisent simultanément lesdites images (20) et ledit modèle (50) sélectionnés en superposant ledit modèle (50) auxdites images (20) à chaque instant.

5. Dispositif (1) selon au moins une revendication précédente, dans lequel ledit processeur (4) adapte, à tout moment, ledit modèle (50) auxdites images (20) en déformant ledit modèle (50) de telle sorte que lesdits points de localisation (23, 51) coïncident et que lesdits moyens d'affichage (3) reproduisent ledit modèle (50) adapté, à tout moment, auxdites images (20).

6. Dispositif (1) selon au moins une revendication précédente, dans lequel lesdits points de localisation correspondent à des repères.

7. Dispositif (1) selon au moins une revendication précédente, dans lequel lesdits moyens d'acquisition (2) acquièrent une pluralité d'images au même moment et à la même longueur d'onde que lesdits signaux d'acquisition de manière à reconstruire ladite partie du corps en trois dimensions.

8. Procédé destiné à afficher des structures de sous-surface comprenant :
- l'acquisition (4a) d'une pluralité d'images (20) d'une partie du corps d'un utilisateur (10) ou d'un objet au moyen de signaux d'acquisition définissant une bande de fréquence multispectrale,
- le traitement (4b) desdites images (20) pour extraire des signaux de référence comprenant de premiers points de localisation (23) de surface et/ou de sous-surface définis par ladite partie dudit corps ou dudit objet,
- la comparaison (4c) desdites images (20) avec une pluralité de modèles (50) de structures de sous-surface de ladite partie dudit corps ou dudit objet, chacun définissant des configurations prédéterminées de seconds points de localisation (51),
- la sélection (4c) dudit modèle (50) dans lequel lesdits seconds points de localisation (51) correspondent davantage auxdits premiers points de localisation (23),
et comprenant :
- l'affichage (3a) d'au moins l'une desdites images (20) et dudit modèle (50) sélectionné de telle sorte que ledit opérateur puisse voir, en temps réel, la structure de sous-surface de ladite partie dudit corps ou dudit objet,
- lesdits modèles (50) étant des modèles anatomiques prédéterminés comprenant, par exemple, des muscles faciaux, ou des veines faciales, ou des nerfs de manière à définir différentes configurations à différentes profondeurs potentiellement de la même partie du corps, et étant tridimensionnels et chacune desdites images (20) est acquise à une longueur d'onde respective desdits signaux d'acquisition, les longueurs d'onde respectives de chacune de chacune desdites images étant différentes les unes des autres, de manière à produire, en reconstruisant un vecteur déterminé par toutes lesdites images, des signaux de référence tridimensionnels définissant une cartographie de ladite partie dudit corps.

9. Procédé selon au moins la revendication 8, dans lequel pendant ou avant ladite étape d'affichage (3a), ledit processeur (4) adapte (4d), moment par moment, le modèle (50) sélectionné auxdites images (20) en déformant le modèle (50) sélectionné de manière à ce que lesdits seconds points de localisation (51) coïncident avec lesdits premiers points de localisation (23), et superpose (4e) le modèle (50) sélectionné sur au moins une dite image (20) de manière à afficher (3a) ledit modèle (50) et ladite image (20) mutuellement combinés l'un avec l'autre.
